# EUROPEAN PATENT APPLICATION

(11) **EP 4 403 165 A1**
(43) Date of publication of application: **24.07.2024**
(21) Application number: 22868773.7
(22) Date of filing: 21.06.2022
(51) Int. Cl.: A61K 9/08, A61K 38/14, A61K 47/18, A61P 31/04

(54) **COMPOSITION OF VANCOMYCIN AQUEOUS SOLUTION**

(30) Priority: 14.09.2021 CN 202111076889
(71) Applicant: Hainan Poly Pharmaceutical Co., Ltd., Haikou, Hainan 571127 (CN); Zhejiang Poly Pharmaceutical Co., Ltd., Hangzhou, Zhejiang 311199 (CN)
(72) Inventor: ZHU, Yifan, Hangzhou, Zhejiang 311199 (CN); FAN, Minhua, Hangzhou, Zhejiang 311199 (CN); LU, Ping, Hangzhou, Zhejiang 311199 (CN); ZHAO, Zhenkun, Hangzhou, Zhejiang 311199 (CN); XU, Cangsu, Hangzhou, Zhejiang 311199 (CN); SHI, Hui, Hangzhou, Zhejiang 311199 (CN); SHEN, Le, Hangzhou, Zhejiang 311199 (CN)
(74) Representative: IPAZ
(86) International application number: PCT/CN2022/100188
(87) International publication number: WO 2023/040396

(57) **Abstract**

The present invention relates to the technical field of medicine, and in particular, relates to a composition of vancomycin aqueous solution. The pharmaceutical composition contains vancomycin or a pharmaceutically acceptable salt thereof, N-methylalanine, and water. The stability of vancomycin in the state of an aqueous solution can be improved by using N-methylalanine.

## Description

### FIELD OF THE INVENTION

The invention relates to the field of medical treatments, and particularly relates to a composition of vancomycin aqueous solution.

### BACKGROUND OF THE INVENTION

The chemical name of Vancomycin hydrochloride is (3S-(3R*,6S*(S*),7S*,225*,23R*,26R*,365*,38aS*))-3-(2-Amino-2-oxoethyl)-44-((2-O-(3-amino-2, 3,6-trideoxy-3-C-methyl-alpha-L-lyxo-hexopyranosyl)-beta-D-glucopyranosyl)oxy)-10,19-dichloro-2, 3,4,5,6,7,23,24,25,26,36,37,38,38a-tetradecahydro-7,22,28,30,32-pentahydroxy-6-((4-methyl-2-(methy lamino)-1-oxopentyl)amino)-2,5,24,38,39-pentaoxo-22H-8,11:18,21-dietheno-23,36-(iminomethano)-13,16:31,35-dimetheno-1H,16H-(1,6,9)oxadiazacyclohexadecino(4,5-m)(10,2,16)benzoxadiazacyclote tracosine-26-carboxylic acid, with a molecular weight of 1485.7 and a molecular formula of C₆₆H₇₆Cl₃N₉O₂₄. Vancomycin hydrochloride has the following structural formula:

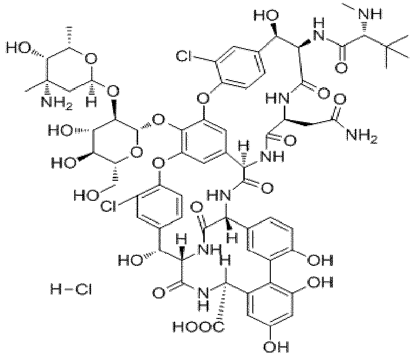

Vancomycin hydrochloride is a glycopeptide antibiotic from Streptomyces orientalis or Nocardiaspp.. The action mechanism of vancomycin hydrochloride is a high-affinity binding to the alanylalanine, the terminal of the precursor peptide of the sensitive bacterial cell wall, so as to block the synthesis of polymeric peptidoglycans constituting the bacterial cell wall, and to hinder the synthesis of the bacterial cell wall. In addition, vancomycin may also change bacterial cell membrane permeability and selectively inhibit the synthesis of RNA.

Vancomycin hydrochloride is characterized by strong bactericidal effect only on gram-positive bacteria, and bacteria do not generate resistance to the product, and there is no cross-resistance with other antibiotics; the main clinical application is infection caused by methicillin-resistant Staphylococcus aureus (MRSA), methicillin-resistant coagulase-negative Staphylococcus (MRCNS), and penicillin-resistant Streptococcus pneumoniae (PRSP). It is a preferred drug for infection caused by methicillin-resistant Staphylococcus aureus, methicillin-resistant coagulase-negative staphylococci and the like.

However, the stability of vancomycin hydrochloride is poor, especially in the state of an aqueous solution. It can be observed that the solution color gradually turns red at normal temperature, which may be due to the existence of phenolic group and diphenol group in the molecular structure of vancomycin, and the phenolic aqueous solution is easily hydrated or oxidized into quinone when it is exposed to light, and thus becomes red.

At the same time, the structure of vancomycin is composed of a heptapeptide containing an asparagine, which is prone to degradation in aqueous solutions. In the state of aqueous solution, the asparagine side chain in the structure is prone to deamination, hydrolysis and rearrangement reaction, resulting in an unstable prescription. The major degradation impurities of vancomycin is Des-(amido)-succinimide-vancomycin B (RS2), CDP-1major ((1.2M)-[L-β-Asp³] vancomycin B, CDP-IM) and CDP-1 minor ([L-β-Asp³] vancomycin B, CDP-Im). 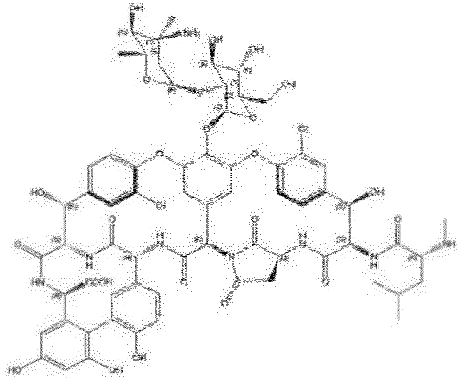 Des-(amido)-succinimide-vancomycin B (RS2) 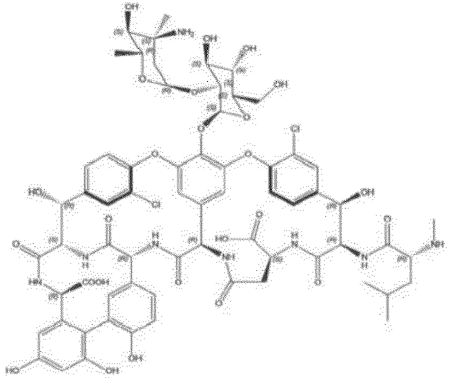 CDP-1m ([L-β-Asp³] vancomycin B, CDP-I minor) 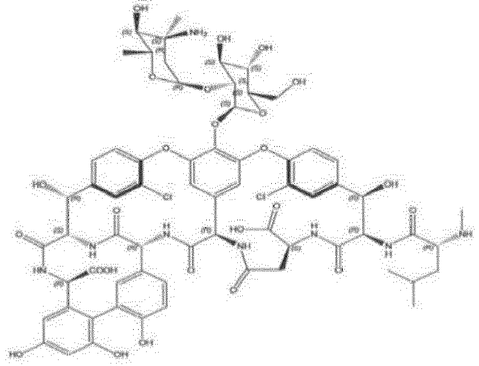 CDP-1M ((1.2M)-[L-β-Asp³] vancomycin B, CDP-I major)

The marketed dosage forms of vancomycin hydrochloride include lyophilized powder or frozen solution for injection, dry mixtures and capsules for oral administration, and ophthalmic vancomycin ointments (oily base, anhydrous). Vancomycin mostly presents in anhydrous form. The preparation of vancomycin injection, vancomycin eye drops, vancomycin external gel/emulsion/spray for direct use creates an enormous challenge due to the stability of vancomycin aqueous solution, which limits the development and use of other dosage forms of vancomycin.

The existing vancomycin freeze-dried powder injection needs to be dispersed and diluted before use, and the use steps are tedious; although a vancomycin injection has been developed by Baxter, cryopreservation is required, and the vancomycin injection is taken out and thawed it into solution before use, which is very inconvenient. Moreover, due to stability issues, the diluted vancomycin injection should be used as soon as possible to prevent the formation of impurities and visible particles after prolonged storage.

Staphylococcus aureus, especially methicillin-resistant Staphylococcus aureus, is a main pathogenic bacterium leading to traumas, chronic wounds, and surgical site infection (SSI). Vancomycin can effectively inhibit MRSA, and therefore it is expected to be an effective drug for the treatment of MRSA infection of traumas and chronic wounds in ocular, ear and other parts, as well as preventing and reducing the risk of MRSA infection in surgical sites. However, the stability of vancomycin aqueous solution limits the development of vancomycin ophthalmic formulations and vancomycin topical gels/emulsions/sprays. Although ophthalmic vancomycin ointments are available, they are inconvenient for administration by adopting an oily base (Vaseline, liquid paraffin), and the line of sight is affected after administration.

Therefore, it is necessary to achieve stability of vancomycin in aqueous solution. A stable vancomycin solution makes it possible to develop ready-to-use vancomycin injections, ophthalmic formulations, ear formulations, nasal formulations, etc., which is beneficial to reducing the working intensity of medical staff, reducing the safety risk caused by stability, improving the use convenience, expanding the application range of the vancomycin, and better exerting the efficacy of vancomycin.

### SUMMARY OF INVENTION

In order to solve the problems in the prior art, the invention provides a pharmaceutical composition comprising vancomycin or a pharmaceutically acceptable salt thereof, N-methylalanine, and water.

The stability of vancomycin in aqueous solution can be improved by using N-methylalanine. Preferably, in the pharmaceutical composition:
a concentration of the vancomycin or a pharmaceutically acceptable salt thereof is 0.1-30% w/w, more preferably 0.1-15% w/w;
a concentration of the N-methylalanine is 0.1-30% w/w, a concentration of the N-methylalanine is 0.1-30% w/w, more preferably 0.1-15% w/w.

Preferably, the pH of the composition is 3-9, more preferably, the pH of the composition is 3-6, and the pH can be adjusted with an acid or a base. The examples are, but not limited to, hydrochloric acid, acetic acid and sodium hydroxide.

The N-methylalanine is N-methyl-DL-alanine, N-methyl-D-alanine or N-methyl-L-alanine. More preferably, the N-methylalanine is N-methyl-D-alanine.

Preferably, the molar ratio of N-methyl-amino acid to vancomycin is (0.1-40):1, more preferably, the mole ratio of N-methyl-amino acid to vancomycin is (0.5-20):1.

In one embodiment of the invention, the composition may comprise a pH regulator, an ion chelating agent, an osmotic pressure regulator, a preservative, etc.

Illustratively, the pH regulator includes, but is not limited to one or more of acetic acid, sodium acetate, succinic acid, amino acid, malic acid, lactic acid, citric acid, sodium citrate, sodium dihydrogen phosphate, disodium hydrogen phosphate, phosphoric acid and boric acid.

Illustratively, the ion chelating agent includes, but is not limited to one or more of disodium ediate, citric acid, etc.

Illustratively, the preservative includes, but is not limited to one or more of benzalkonium chloride, cresol, phenol, p-hydroxybenzoate, benzyl alcohol, EDTA, etc.

Illustratively, the osmotic pressure regulator includes, but is not limited to sodium chloride, glucose, etc.

Another object of the invention is to provide the use of a pharmaceutical composition for the manufacture of a medicament for treating or preventing bacterial infections including a staphylococcal infection.

Preferably, the bacterial infection is caused by a methicillin-resistant Staphylococcus strain (MRSA).

The vancomycin in the pharmaceutical composition of the invention can exist stably after 10 days under the condition of 25°C/60% RH.

The pharmaceutical composition of the invention can well improve the stability of the vancomycin gel prescription and remain stable at least within 30 days under the condition of 25°C/60% RH.

The pharmaceutical composition of the invention can well improve the stability of the vancomycin eye drop prescription and remain stable at least within 30 days under the condition of 25°C/60% RH.

The pharmaceutical composition of the invention can well improve the stability of the vancomycin injection prescription and remain stable at least within 30 days under the condition of 25°C/60% RH.

The vancomycin solution formulation of the invention can exhibit the same antibacterial activity as the vancomycin aqueous solution with the same vancomycin concentration and without N-methyl-alanine.

The pharmaceutical composition of the invention can be administered in an undiluted or diluted form prior to administration. It may be diluted with a 5% or 10% dextrose solution or another injectable diluent or infusion solution. The route of administration may include, but are not limited to injection, instillation, inhalation, oral, otic, nasal, topical, ocular, vaginal, and rectal administration. The pharmaceutical composition of the invention may be delivered using a needle/syringe, an infusion set, a catheter, an applicator, a bottle, a nebulizer, an inhalation device or as a wound dressing.

The pharmaceutical composition of the invention may be filled in a glass vial, a syringe, a dropper bottle, a tube, an applicator, a unit dispenser, an infusion bag, a nebulizer, an inhalation device or other medication containers. Optionally, the pharmaceutical composition may be filled and protected with an inert gas such as nitrogen.

The pharmaceutical composition of the invention can be used to prepare injections, ophthalmic formulations, otic formulations, nasal formulations, and the like.

The aqueous solution of the vancomycin pharmaceutical composition provided by the invention is stable under a non-freezing condition, and the physical and chemical degradation of vancomycin can be effectively inhibited due to the existence of the N-methyl-alanine. The vancomycin aqueous solution provided by the invention has excellent potential in developing ready-to-use vancomycin injections, ophthalmic formulations, otic formulations, nasal formulations, and the like.

### DESCRIPTION OF FIGURE

Figure 1 is the HPLC profile of the detection result after prescription F-4 was placed under the condition of 25°C/60% RH for 10 days.
Figure 2 is the HPLC profile of the detection result after prescription F-20 was placed under the condition of 25°C/60% RH for 10 days.
Figure 3 is the HPLC profile of the detection result after prescription F-25 was placed under the condition of 25°C/60% RH for 30 days.
Figure 4 is the HPLC profile of the detection result after prescription F-29 was placed under the condition of 25°C/60% RH for 30 days.
Figure 5 is the HPLC profile of the detection result after prescription F-32 was placed under the condition of 25°C/60% RH for 30 days.

### EMBODIMENTS

In order to better understand the technical solution of the invention, the invention is further illustrated by following embodiments. The embodiments are intended to facilitate understanding of the invention and should not be deemed as a limitation to the invention.

HPLC analysis conditions of the following embodiments were:
Chromatographic column: Welch Ultimate XB-C18 (250×4.6 mm, 5 µm)
Detection wavelength: 280 nm;
Column temperature: 30°C;
Flow rate: 2.0 mL/min;
Injection volume: 20 µL;
Mobile phase:
Solution A: 4.0 mL of triethylamine was precisely measured, and water was added to bring the total volume to 2000 mL, and the pH of the solution was further adjusted to 3.20 with phosphoric acid;
Solution B: Acetonitrile-tetrahydrofuran-solution A = 55-10-935, the pH was adjusted to 3.20 with phosphoric acid;
Solution C: acetonitrile-tetrahydrofuran-solution A = 290-10-700, the pH was adjusted to 3.20 by phosphoric acid;

Elution gradient:

| Time | Solution B(%) | Solution C(%) |
|---|---|---|
| 0 | 100 | 0 |
| 12 | 100 | 0 |
| 20 | 0 | 100 |
| 23 | 100 | 0 |
| 32 | 100 | 0 |

### Embodiment 1: Effect of different amino acids on the stability of vancomycin

An aqueous solution of vancomycin hydrochloride was prepared, wherein the concentration of vancomycin hydrochloride was 5% w/w and the concentration of stabilizer (amino acid) was 0.25% w/w. After the solution was placed under the condition 25 °C / 60%RH for 10 days, relevant substances were performed by HPLC. Different stabilizers (amino acids) were applied in the composition, and corresponding test result is listed in Table 1.

**Table 1**

| % w/w | F-1 | F-2 | F-3 | F-4 | F-5 | F-6 | F-7 | F-8 | F-9 | F-10 | F-11 | F-12 | F-13 | F-14 | F-15 | F-16 | F-17 |
|---|---|---|---|---|---|---|---|---|---|---|---|---|---|---|---|---|---|
| Vancomycin hydrochloride | 5% | 5% | 5% | 5% | 5% | 5% | 5% | 5% | 5% | 5% | 5% | 5% | 5% | 5% | 5% | 5% | 5% |
| L-Tryptophan | | 0.67 | | | | | | | | | | | | | | | |
| L-Phenylalani ne | | | 0.67 | | | | | | | | | | | | | | |
| DL-Alanine | | | | 0.67 | | | | | | | | | | | | | |
| L-serine | | | | | 0.67 | | | | | | | | | | | | |
| L-Leucine | | | | | | 0.67 | | | | | | | | | | | |
| Isoleucine | | | | | | | 0.67 | | | | | | | | | | |
| D-Proline | | | | | | | | 0.67 | | | | | | | | | |
| L-Proline | | | | | | | | | 0.67 | | | | | | | | |
| methionine | | | | | | | | | | 0.67 | | | | | | | |
| L-Threonine | | | | | | | | | | | 0.67 | | | | | | |
| L-Cysteine | | | | | | | | | | | | 0.67 | | | | | |
| L-lysine | | | | | | | | | | | | | 0.67 | | | | |
| L-Arginine | | | | | | | | | | | | | | 0.67 | | | |
| L-Histidine | | | | | | | | | | | | | | | 0.67 | | |
| 4-Aminobutyr ic acid | | | | | | | | | | | | | | | | 0.67 | |
| DL-Glycine | | | | | | | | | | | | | | | | | 0.67 |
| After the compositions were placed under the condition of 25°C/60%RH for 10 days, CDP-1M, CDP-1m and total impurities were detected. | | | | | | | | | | | | | | | | | |
| RS2 | 3.43 | 1.89 | 2.68 | 1.87 | 3.21 | 3.12 | 3.17 | 3.17 | 3.09 | 3.13 | 3.21 | 3.16 | 1.11 | 2.08 | 1.44 | 3.63 | 3.21 |
| CDP-1 | 0.75 | 0.73 | 0.73 | 0.76 | 0.77 | 0.75 | 0.76 | 0.75 | 0.75 | 0.74 | 0.77 | 0.73 | 2.34 | 2.38 | 1.57 | 1.32 | 0.76 |
| Vancomycin HCl | 91.0 8 | 92.9 5 | 91.5 1 | 93.2 4 | 91.1 2 | 91.3 1 | 91.25 | 91.2 5 | 91.2 6 | 91.2 4 | 91.00 | 88.79 | 87.85 | 87.7 2 | 91.0 8 | 89.66 | 91.4 1 |
| Total impurities | 8.92 | 7.05 | 8.49 | 6.76 | 8.88 | 8.69 | 8.75 | 8.75 | 8.74 | 8.76 | 9.00 | 11.21 | 12.15 | 12.2 8 | 8.92 | 10.34 | 8.59 |

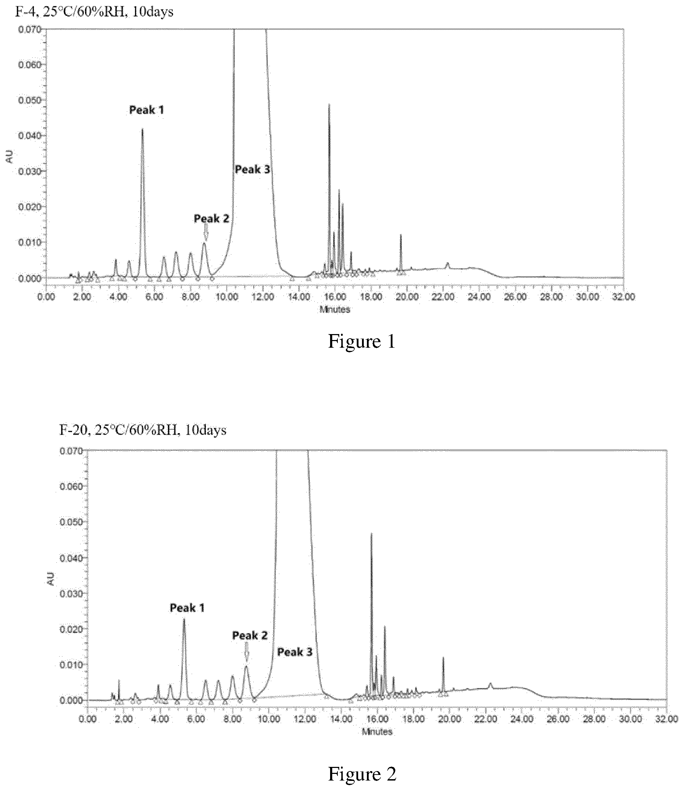

It was found that alanine could slow down the degradation of vancomycin. Other amino acids were less effective, or accelerated degradation. The corresponding data of HPLC profile (Figure 1) of F-4 (DL-alanine) is shown in Table 2.

**Table 2**

| Peak | Name | R RT min | Area% | Area |
|---|---|---|---|---|
| 1 | RS2 | 5.335 | 1.87 | 470871 |
| 2 | CDP-1 | 8.749 | 0.76 | 191171 |
| 3 | Vancomycin HCL | 10.589 | 93.24 | 23585597 |

### Embodiment 2: Effect of N-methyl-alanine on the stability of vancomycin

The experimental method was as described in Embodiment 1, the stabilizers (amino acids) and the corresponding test results are listed in Table 3.

**Table 3**

| % w/w | F-18 | F-19 | F-20 | F-21 | F-22 | F-23 |
|---|---|---|---|---|---|---|
| Vancomycin hydrochloride | 5% | 5% | 5% | 5% | 5% | 5% |
| DL-2-aminobutyric acid | | 0.68 | | | | |
| N-methyl-DL-alanin e | | | 0.67 | | | |
| L-alanyl-L-glutamin e | | | | 1.46 | | |
| N-methyl-D-alanine | | | | | 0.67 | |
| N-methyl-L-alanine | | | | | | 0.67 |
| After the compositions were placed under the condition of 25°C/60%RH for 10 days, CDP-1M, CDP-1m and total impurities were detected. | | | | | | |
| RS2 | 3.53 | 1.67 | 1.21 | 1.13 | 0.71 | 2.9 |
| CDP-1 | 0.69 | 0.85 | 0.71 | 0.92 | 0.41 | 0.77 |
| Vancomycin HCl | 91.09 | 91.91 | 94.66 | 90.18 | 95.15 | 91.8 |
| Total impurities | 8.91 | 8.09 | 5.34 | 9.82 | 4.85 | 8.2 |

N-methyl-DL-alanine is a derivative of alanine, and its stabilization effect on vancomycin is superior to alanine. The effect of N-methyl-D-alanine is especially prominent. The corresponding data of HPLC profile (Figure 2) of F-20 (N-methyl-DL-alanine) is shown in Table 4.

**Table 4**

| Peak | Name | R RT min | Area% | Area |
|---|---|---|---|---|
| 1 | RS2 | 5.332 | 1.12 | 280162 |
| 2 | CDP-1 | 8.762 | 0.71 | 177973 |
| 3 | Vancomycin HCL | 10.576 | 94.66 | 23824054 |

### Embodiment 3: Effect of N-methyl-alanine on the stability of vancomycin gel

Weighting a prescription amount of N-methyl-alanine in a beaker, adding a prescription amount of water and acetic acid, and stirring to make the mixture uniform; weighting a prescription amount of gelatin, adding it into the above solution, stirring to dissolve; weighting a prescription amount of vancomycin hydrochloride, and stirring to dissolve. After being placed under accelerated conditions of 25°C/60%RH for 30 days, relevant substances were sampled and detected. The result is shown in Table 5, the percentage of impurities peak area is calculated by peak area normalization method.

**Table 5**

| % w/w | 0 day | F-24 | F-25 | F-26 | F-27 |
|---|---|---|---|---|---|
| Vancomycin hydrochloride | / | 1% | 1% | 1% | 1% |
| N-methyl-DL-alanin e | / | | 0.33 | 0.17 | |
| N-methyl-D-alanine | / | | | | 0.33 |
| Gelatine | / | 0.75% | 0.75% | 0.75% | 0.75% |
| Acetic acid | / | 1.0% | 1.0% | 1.0% | 1.0% |
| After the compositions were placed under the condition of 25°C/60%RH for 30 days, CDP-1M, CDP-1m and total impurities were detected. | | | | | |
| RS2 | 0.55 | 2.03 | 0.22 | 1.33 | 0.65 |
| CDP-1 | 0.32 | 0.99 | 0.40 | 0.69 | 0.31 |
| Vancomycin HCl | 95.82 | 89.42 | 95.28 | 94.07 | 95.77 |
| Total impurities | 4.18 | 10.58 | 4.72 | 5.93 | 4.23 |

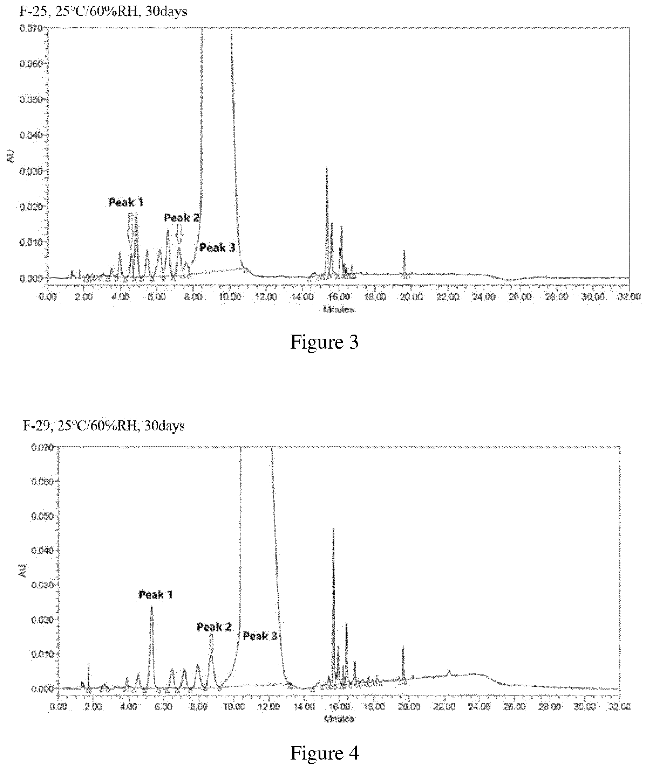

The experimental result shows that N-methyl-D-alanine could well improve the stability of vancomycin gelatine prescription under the condition of 25°C/60%RH and could remain stable at least within 30 days. After F-25 prescription was placed under the conditions of 25°C/60%RH for 30 days, and relevant substances were sampled and detected, the HPLC profile (Figure 3) of the detection result is shown in Table 6.

**Table 6**

| Peak | Name | R RT min | Area% | Area |
|---|---|---|---|---|
| 1 | RS2 | 4.594 | 0.219 | 58915 |
| 2 | CDP-1 | 7.222 | 0.402 | 108293 |
| 3 | Vancomycin HCL | 8.631 | 95.28 | 25694407 |

### Embodiment 4: Effect of N-methyl-alanine on the stability of vancomycin eye drops

Weighting a prescription amount of N-methyl-alanine in a beaker, adding a prescription amount of water, and stirring to make the mixture uniform; weighting a prescription amount of benzalkonium chloride and glycine, adding them into the above solution, stirring to dissolve; weighting a prescription amount of vancomycin hydrochloride, and stirring to dissolve. After being placed under accelerated conditions of 25°C/60%RH for 30 days, relevant substances were sampled and detected. The result is shown in Table 7, the percentage of impurities peak area is calculated by peak area normalization method.

**Table 7**

| % w/w | 0 day | F-28 | F-29 | F-30 |
|---|---|---|---|---|
| Vancomycin hydrochloride | / | 3% | 3% | 3% |
| N-methyl-DL-alanine | / | | 1.0% | |
| N-methyl-D-alanine | / | | | 1.0% |
| benzalkonim chloride | / | 0.01% | 0.01% | 0.01% |
| glycine | / | 1.0% | 1.0% | 1.0% |
| Adjusting the pH to 5.0±0.2 with 0.5 M HCl solution or sodium hydroxide solution; | | | | |
| After the compositions were placed under the condition of 25°C/60%RH for 30 days, CDP-1M, CDP-1m and total impurities were detected. | | | | |
| RS2 | 0.53 | 2.18 | 1.20 | 0.60 |
| CDP-1 | 0.32 | 0.89 | 0.72 | 0.33 |
| Vancomycin HCl | 95.81 | 89.7 | 94.63 | 95.7 |
| Total impurities | 4.19 | 10.30 | 5.37 | 4.30 |

The experimental result shows that N-methyl-D-alanine could well improve the stability of vancomycin eye drops prescription and could remain stable at least within 30 days under the condition of 25°C/60% RH. After F-29 prescription was placed under the conditions of 25°C/60%RH for 30 days, and relevant substances were sampled and detected, the HPLC profile (Figure 4) of the detection result is shown in Table 8.

**Table 8**

| Peak | Name | R RT min | Area% | Area |
|---|---|---|---|---|
| 1 | RS2 | 5,304 | 1.20 | 295178 |
| 2 | CDP-1 | 8.707 | 0.72 | 176521 |
| 3 | Vancomycin HCL | 10.558 | 94.63 | 23334406 |

### Embodiment 5: Effect of N-methyl-alanine on the stability of vancomycin injection

Weighting a prescription amount of N-methyl-alanine in a beaker, adding a prescription amount of water, and stirring to make the mixture uniform; weighting a prescription amount of polyethylene glycol 400 and L-lysine hydrochloride, adding them into the above solution, stirring to dissolve; weighting a prescription amount of vancomycin hydrochloride, and stirring to dissolve. After being placed under accelerated conditions of 25°C/60%RH for 30 days, relevant substances were sampled and detected. The result is shown in Table 9, the percentage of impurities peak area is calculated by peak area normalization method.

**Table 9**

| % w/w | 0 day | F-31 | F-32 | F-33 |
|---|---|---|---|---|
| Vancomycin hydrochloride | / | 0.5% | 0.5% | 0.5% |
| N-methyl-DL-alanine | / | | 1.0% | |
| N-methyl-D-alanine | / | | | 1.0% |
| Polyethylene glycol 400 | / | 1.8% | 1.8% | 1.8% |
| L-Lysine hydrochloride | / | 1.26% | 1.26% | 1.26% |
| Adjusting the pH to 5.0±0.2 with 0.5 M HCl solution or sodium hydroxide solution; | | | | |
| After the compositions were placed under the condition of 25°C/60%RH for 30 days, CDP-1M, CDP-1m and total impurities were detected. | | | | |
| RS2 | 0.55 | 2.97 | 1.15 | 0.60 |
| CDP-1 | 0.32 | 0.92 | 0.72 | 0.32 |
| Vancomycin HCl | 95.19 | 87.81 | 94.62 | 95.72 |
| Total impurities | 4.19 | 12.19 | 5.38 | 4.28 |

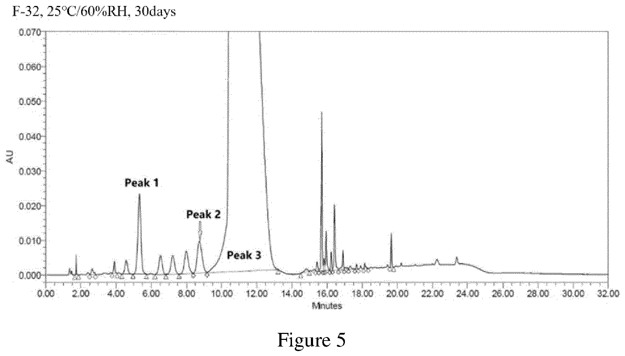

The experimental result shows that N-methyl-D-alanine could improve the stability of vancomycin injection prescription and could remain stable at least within 30 days under the condition of 25°C/60% RH. After F-32 prescription was placed under the conditions of 25°C/60%RH for 30 days, and relevant substances were sampled and detected, the HPLC profile (Figure 5) of the detection result is shown in Table 10.

**Table 10**

| Peak | Name | R RT min | Area% | Area |
|---|---|---|---|---|
| 1 | RS2 | 5.336 | 1.15 | 283427 |
| 2 | CDP-1 | 8.731 | 0.72 | 177649 |
| 3 | Vancomycin HCL | 10,565 | 94.62 | 23387061 |

### Embodiment 6: PK test of vancomycin injection

Nine male SD rats were divided into three groups. The first group (G1) were once intravenously injected with vancomycin (5 mg/mL) dissolved in physiologica saline, the second group (G2) were once intravenously injected with vancomycin reference preparation (Xellia Company, batch number 1V14003), the third group 3 (G3) were once intravenously injected with homemade vancomycin injection (50mg/mL vancomycin, 53.4mg/mL N-methyl-D-alanine), the dosages administered in the three groups were consistently 20 mg/kg. Plasma samples were collected at 0.083, 0.167, 0.25, 0.5, 1, 2, 4, 6, 8, 12, and 24 h post-dosing in each group. The concentration of vancomycin in plasma samples was detected using an established LC-MS/MS method.

The result showed that the elimination half-life (T1/2) of the vancomycin in SD rats following a single intrathecal dose was 0.678-0.842 h, and the blood circulation time was short. The apparent volume of distribution (Vd) was significantly larger than the whole blood volume of rats, which indicated that the vancomycin had widely distributed throughout the body. Comparisons between the pharmacokinetic parameters in G1, G2, and G3 showed that, the pharmacokinetic behaviors in G1, G2, and G3 were basically the same, and there were no significant difference.

| Parameter | Unit | G1 | G2 | G3 |
|---|---|---|---|---|
| AUC ₍₀₋ₜ₎ | µg/mL*h | 59.7 | 60.5 | 74.1 |
| AUC_{(0-∞)} | µg/mL*h | 61.8 | 61.4 | 75.4 |
| T_{1/2} | h | 0.842 | 0.678 | 0.694 |
| Tₘₐₓ | h | 0.083 | 0.083 | 0.083 |
| MRT_{(0-∞)} | h | 1.01 | 0.880 | 0.827 |
| Cₘₐₓ | µg/mL | 83.8 | 68.9 | 89.5 |
| Vd | L/kg | 0.393 | 0.319 | 0.266 |
| Cl | L/h/kg | 0.329 | 0.326 | 0.267 |

## Claims

1. A pharmaceutical composition comprising vancomycin or a pharmaceutically acceptable salt thereof, N-methylalanine, and water.

2. The pharmaceutical composition according to claim 1, wherein the concentration of the vancomycin or a pharmaceutically acceptable salt thereof is 0.1-30% w/w.

3. The pharmaceutical composition according to claim 1, wherein the concentration of the vancomycin or a pharmaceutically acceptable salt thereof is 0.1-15% w/w.

4. The pharmaceutical composition according to claim 1, wherein the concentration of the N-methyl-D-alanine is 0.1-30% w/w.

5. The pharmaceutical composition according to claim 1, wherein the concentration of the N-methyl-D-alanine is 0.1-15% w/w.

6. The pharmaceutical composition according to claim 1, wherein the N-methylalanine is N-methyl-DL-alanine, N-methyl-D-alanine or N-methyl-L-alanine.

7. The pharmaceutical composition according to claim 1 with a pH between 3 and 9.

8. The pharmaceutical composition according to claim 7 with a pH between 3 and 6.

9. The pharmaceutical composition according to claim 7 or claim 8, wherein the pH can be adjusted with an acid or a base.

10. The pharmaceutical composition according to claim 9, wherein the acid is hydrochloric acid, acetic acid, and the base is sodium hydroxide.

11. The pharmaceutical composition according to claim 1, wherein the N-methylalanine is N-methyl-D-alanine.

12. The pharmaceutical composition according to any one of claims 1-11, wherein the molar ratio of N-methyl-amino acid to vancomycin is (0.1-40):1.

13. The pharmaceutical composition according to claim 12, wherein the molar ratio of N-methyl-amino acid to vancomycin is (0.5-20):1.

14. Use of the pharmaceutical composition according to any one of claims 1-13 for the manufacture of a medicament for the treatment or prevention of bacterial infections including a staphylococcal infection and Streptococcus pneumoniae.

15. The use of the pharmaceutical composition according to claim 14, wherein the bacterial infection is caused by methicillin-resistant staphylococcus aureus (MRSA), methicillin-resistant coagulase-negative staphylococcus or penicillin-resistant streptococcus pneumoniae (PRSP).
